(19) Europäisches Patentamt
European Patent Office
Office européen des brevets

(11) **EP 2 440 266 B1**

(12) **EUROPÄISCHE PATENTSCHRIFT**

(45) Veröffentlichungstag und Bekanntmachung des Hinweises auf die Patenterteilung:
**23.04.2014   Patentblatt 2014/17**

(21) Anmeldenummer: **10728117.2**

(22) Anmeldetag: **01.06.2010**

(51) Int Cl.:
**A61M 1/36** (2006.01)

(86) Internationale Anmeldenummer:
**PCT/EP2010/003329**

(87) Internationale Veröffentlichungsnummer:
**WO 2010/142394 (16.12.2010 Gazette 2010/50)**

(54) **Vorrichtung zur Überwachung eines Flüssigkeitssystems einer extrakorporalen Blutbehandlungsvorrichtung**

Device for monitoring a fluid system of an extracorporeal blood treatment device

Dispositif de surveillance d'un système à liquide d'un dispositif de traitement extracorporel du sang

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **09.06.2009   DE 102009024864**

(43) Veröffentlichungstag der Anmeldung:
**18.04.2012   Patentblatt 2012/16**

(73) Patentinhaber: **Fresenius Medical Care Deutschland GmbH**
**61352 Bad Homburg v.d.H. (DE)**

(72) Erfinder:
• KOPPERSCHMIDT, Pascal
  97456 Dittelbrunn (DE)
• NÜRNBERGER, Thomas
  97705 Burkardroth (DE)

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
**EP-A1- 0 330 761     US-A1- 2009 071 911**

**Beschreibung**

Die Erfindung bezieht sich auf eine Vorrichtung zur Überwachung eines Flüssigkeitssystems einer extrakorporalen Blutbehandlungsvorrichtung, die einen extrakorporalen Blutkreislauf mit einer arteriellen Blutleitung, die zu einer ersten Kammer eines durch eine semipermeable Membran in die erste Kammer und eine zweite Kammer unterteilten Dialysators oder Filters führt, und einer venösen Blutleitung aufweist, die von der ersten Kammer des Dialysators oder Filters abgeht. Darüber hinaus betrifft die Erfindung eine extrakorporale Blutbehandlungsvorrichtung mit einer Vorrichtung zur Überwachung des Flüssigkeitssystems.

Der extrakorporale Blutkreislauf der bekannten Blutbehandlungsvorrichtungen umfasst neben der arteriellen und venösen Blutleitung sowie der Blutkammer des Dialysators oder Filters eine Einrichtung zum Abscheiden von Luft, die allgemein auch als Luftabscheider bezeichnet wird. Der Luftabscheider weist eine Kammer auf, die von Blut durchflossen wird. Dabei ist die Kammer des Luftabscheiders nicht vollständig mit Blut gefüllt. Mit einer Überwachungseinrichtung wird überwacht, ob die Kammer mit Blut gefüllt ist. Als Luftabscheider finden die bekannten Tropfkammern Verwendung, die stromab des Dialysators oder Filters in der venösen Blutleitung angeordnet sind.

Bei den bekannten Blutbehandlungsvorrichtungen zweigt im Allgemeinen von der venösen Tropfkammer eine Leitung ab, über die das Flüssigkeitssystem der Blutbehandlungsvorrichtung beim Befüllen des extrakorporalen Blutkreislaufs belüftet werden kann. Während der Blutbehandlung ist die Belüftungsleitung verschlossen.

Während der extrakorporalen Blutbehandlung besteht grundsätzlich die Gefahr, dass der Blutspiegel in der venösen Tropfkammer soweit ansteigt, dass Blut in die Belüftungsleitung gelangt. Als Sicherheitsmaßnahme ist in der Belüftungsleitung mindestens ein hydrophober Filter angeordnet, der zwar für Flüssigkeit undurchlässig, für Luft aber durchlässig ist, soweit der Filter nicht mit Flüssigkeit benetzt ist.
Der Hydrophobfilter kann das Blut also wirkungsvoll zurückhalten, selbst wenn Blut in die Belüftungsleitung gelangen sollte. Nachteilig ist jedoch, dass der Filter nach der Benetzung mit Blut gereinigt oder ausgetauscht werden muss, was eine Unterbrechung der Blutbehandlung zur Folge hat und einen erheblichen Aufwand darstellt.

Im Vorfeld- oder nach der Dialyse kann der extrakorporale Blutkreislauf gespült werden, beispielsweise mit einer Kochsalzlösung oder Substituat (Permeat der Dialysierflüssigkeit). Auch in diesem Fall besteht die Gefahr, dass Ersatzflüssigkeit aus der venösen Tropfkammer über die Belüftungsleitung bis an den hydrophoben Filter gelangt.

Bei einer Störung könnte ein Eingriff in die Maschinensteuerung vorgenommen werden. Beispielsweise könnte die Blutpumpe angehalten und die venöse Schlauchklemme geschlossen werden. Wenn bei einem Störfall ein Eingriff in die Maschinensteuerung nicht vorgenommen wird, besteht grundsätzlich die Gefahr, dass die Membran des hydrophoben Filters durchbrochen wird. Es ist möglich, dass nur ein Hydrophobfilter vorhanden ist, der sich in der Belüftungsleitung außerhalb oder im weiteren Verlauf auch innerhalb der Maschine befinden kann. Die Gefahr des Eintritts von Flüssigkeit in die Belüftungsleitung kann verringert werden, wenn zwei oder mehrere hydrophobe Filter in der Belüftungsleitung angeordnet sind, die in Serie geschaltet sind. Während der erste Hydrophobfilter, der außerhalb des Gehäuses der Blutbehandlungsvorrichtung angeordnet werden kann, sich noch relativ leicht reinigen oder austauschen lässt, muss für die Reinigung oder den Austausch des zweiten Hydrophobfilters aber das Gehäuse der Blutbehandlungsvorrichtung geöffnet werden.

Die EP 0 330 761 A1 beschreibt ein Verfahren zur Überwachung des Flüssigkeitssystems einer extrakorporalen Blutbehandlungsvorrichtung, bei dem der Druck in der von der venösen Tropfkammer abzweigenden Belüftungsleitung überwacht wird, in der ein Hydrophober Filter angeordnet ist. Der Druck wird in dem der Tropfkammer abgewandten Abschnitt der Belüftungsleitung überwacht. Dabei werden periodische Druckschwankungen überwacht, die von der Blutpumpe erzeugt werden und sich über das Schlauchsystem über den Hydrophobfilter fortpflanzen. Für den Fall, dass Blut in die Belüftungsleitung gelangen sollte, wird die Membran des Hydrophobfilters mit Flüssigkeit benetzt. Dadurch wird die Membran des Hydrophobfilters für Luft undurchlässig, so dass sich die periodischen Druckschwankungen nicht mehr über die Membran des Hydrophobfilters ausbreiten können. Das Ausbleiben der periodischen Druckschwankungen ist daher ein Indikator für einen Störfall.

Sofern die natürlichen durch die Bauart der peristaltischen Blutpumpe hervorgerufenen Druckschwankungen nicht ausreichen sollten, schlägt die EP 0 330 761 A1 vor, die natürlichen Druckschwankungen zu verstärken und künstlich Förderratenschwankungen der Blutpumpe beispielsweise durch eine Änderung der Pumpendrehzahl zu erzeugen.

Der Erfindung liegt die Aufgabe zugrunde, eine Vorrichtung anzugeben, die eine sichere Überwachung des Flüssigkeitssystems einer extrakorporalen Blutbehandlungsvorrichtung erlaubt, um bei einem Störfall zumindest ein Durchbrechen der Membran des hydrophoben Filters sicher verhindern zu können, aber auch schon das Benetzen der Membran des Hydrophofilters ausschließen oder zumindest frühzeitig erkennen zu können. Eine weitere Aufgabe der Erfindung liegt darin, eine extrakorporale Blutbehandlungsvorrichtung mit einer Vorrichtung zur Überwachung des Flüssigkeitssystems bereitzustellen.

Die Lösung dieser Aufgabe erfolgt erfindungsgemäß mit den Merkmalen der Patentansprüche 1 bzw. 2. Bevorzugte Ausführungsformen der Erfindung sind Gegenstand der Unteransprüche.

Bei der erfindungsgemäßen Vorrichtung wird der Druck in dem der venösen Tropfkammer abgewandten Abschnitt der Belüftungsleitung, d.h. in dem Abschnitt, der von der venösen Tropfkammer aus gesehen hinter dem ersten hydrophoben Filter liegt, überwacht, d.h. der Druck wird stromab des mindestens einen hydrophoben Filters gemessen, wobei auf eine Störung geschlossen wird, wenn

Druckschwankungen ausbleiben. Die Belüftungsleitung kann eine einzelne Stichleitung sein. Es ist aber auch möglich, dass die Belüftungsleitung mehrere sich verzweigende Leitungsabschnitte aufweist. Allein entscheidend ist, dass die Belüftungsleitung ein in sich geschlossenes System darstellt.

Eine erste Ausführungsform der erfindungsgemäßen Vorrichtung sieht zur Erzeugung der periodischen Druckschwankungen vor, das in der venösen Blutleitung angeordnete venöse Absperrorgan, bei dem es sich im Allgemeinen um eine venöse Schlauchklemme handelt, alternierend zumindest teilweise zu schließen und zu öffnen, wodurch der Flüssigkeitspegel in der venösen Tropfkammer alternierend ansteigt und absinkt, so dass Druckschwankungen erzeugt werden, die sich bis in die Belüftungsleitung ausbreiten.

Eine alternative besonders bevorzugte Ausführungsform der erfindungsgemäßen Vorrichtung sieht vor, die Druckschwankungen in dem der venösen Tropfkammer abgewandten Abschnitt der Belüftungsleitung, d.h. stromab des mindestens einen hydrophoben Filters, dadurch zu erzeugen, dass die während der Blutbehandlung stromab des mindestens einen Hydrophobfilters verschlossene Belüftungsleitung stromab des mindestens einen Hydrophobfilters, d.h. in dem der venösen Tropfkammer abgewandten Abschnitt der Belüftungsleitung, alternierend belüftet wird, so dass der Druck in der Belüftungsleitung moduliert wird. Bei dieser alternativen Ausführungsform werden also nicht Druckschwankungen überwacht, die im extrakorporalen Blutkreislauf insbesondere von der Blutpumpe erzeugt werden und sich über die Membran des mindestens einen Hydrophobfilter fortpflanzen, sondern es werden Druckschwankungen überwacht, die durch das Belüften in der Belüftungsleitung selbst entstehen.

Bei den bekannten Blutbehandlungsvorrichtungen ist die Belüftungsleitung ohnehin mit einem Belüftungsventil geschlossen, das zur Erzeugung der periodischen Druckschwankungen nur alternierend geöffnet und geschlossen zu werden braucht. Daher ist nur ein verhältnismäßig geringer technischer Aufwand für die Realisierung der Drucküberwachung erforderlich.

Um den Druck in der Belüftungsleitung aufbauen zu können, muss die im extrakorporalen Blutkreislauf angeordnete Blutpumpe im Betrieb und das venöse Absperrorgan zumindest teilweise geschlossen sein. Wenn das Absperrorgan teilweise geschlossen ist, braucht die Blutbehandlung nicht unterbrochen zu werden. Ein vollständiges Schließen des venösen Absperrorgans setzt aber für den Zeitraum der Überwachung eine Unterbrechung der Blutbehandlung voraus. Der Druck in der Belüftungsleitung kann grundsätzlich aber auch mit anderen Mitteln, beispielsweise mit einem Kompressor oder dergleichen aufgebaut werden.

Die Überwachung des Flüssigkeitssystems beruht darauf, dass die Druckschwankungen bei einer Störung nicht mehr feststellbar sind. Hierzu können unterschiedliche Kriterien festgelegt werden. Allein entscheidend ist, dass das Auftreten bzw. Ausbleiben der Druckschwankungen überwacht wird. Derartige Störungen können nicht nur auf durch Flüssigkeit benetzte und dadurch für Luft undurchlässige Hydrophobmembranen (TDPs), sondern beispielsweise auch auf durch Produktionsfehler verklebte und dadurch für Luft undurchlässige Hydrophobmembranen oder nicht angeschlossene Hydrophobmembranen zurückzuführen sein.

Bei einer bevorzugten Ausführungsform wird überprüft, ob ein Druckanstieg feststellbar ist. Ein Druckanstieg kann beispielsweise dadurch festgestellt werden, dass der Druck über einen vorgegebenen Grenzwert ansteigt. Es ist aber auch möglich, die Steigung des gemessenen Drucksignals zu bestimmen, wobei auf einen Druckanstieg geschlossen wird, wenn die Steigung des Messsignals einen vorgegebenen Grenzwert übersteigt. Durch entsprechende Festlegung des Grenzwertes ist es möglich, das Benetzen der Membran des Hydrophobfilters nicht nur auszuschließen, sondern auch frühzeitig zu erkennen.

Wenn bei einer Störung die Schwankungen des Drucksignals ausbleiben, wird vorzugsweise ein akustischer und/oder optischer Alarm gegeben. Vorzugsweise wird im Störfall auch ein Eingriff in die Steuerung der extrakorporalen Blutbehandlungsvorrichtung vorgenommen. Vorzugsweise wird die Blutpumpe angehalten, so dass nicht weiter Blut in die Belüftungsleitung strömen kann.

Im Folgenden werden Ausführungsbeispiele der Erfindung unter Bezugnahme auf die Zeichnungen näher erläutert.

Es zeigen:

Fig. 1     ein erstes Ausführungsbeispiel einer Vorrichtung zur extrakorporalen Blutbehandlung mit einer Vorrichtung zur Überwachung des Flüssigkeitssystems in stark vereinfachter schematischer Darstellung und

Fig. 2     den Verlauf des gemessenen Drucksignals zur Überwachung des Flüssigkeitssystems.

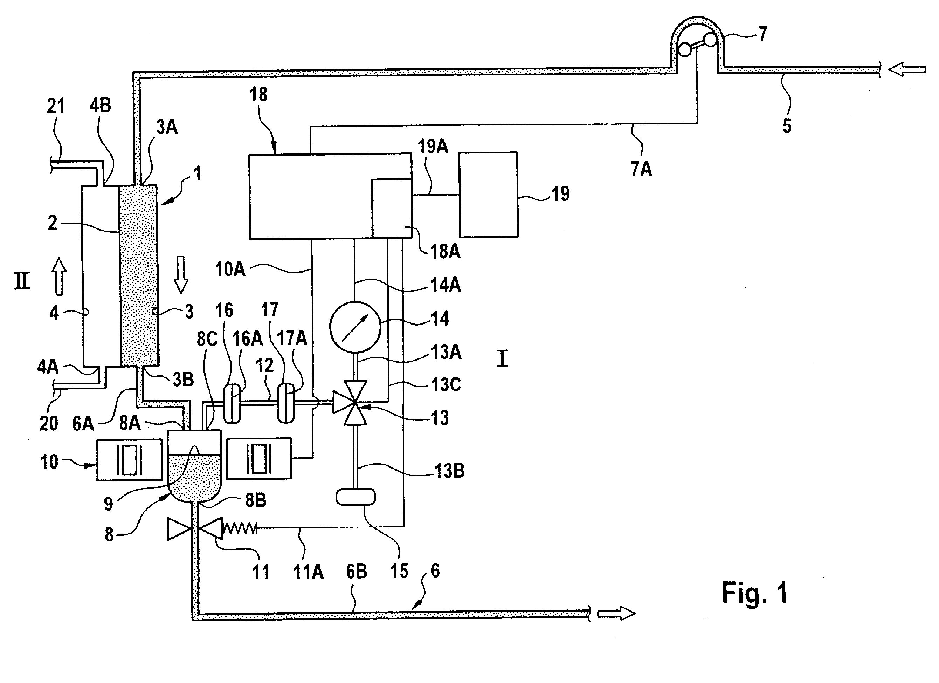

Fig. 1 zeigt die wesentlichen Komponenten eines ersten Ausführungsbeispiels einer extrakorporalen Blutbehandlungsvorrichtung mit einer Vorrichtung zur Überwachung des Flüssigkeitssystems. Bei der extrakorporalen Blutbehandlungsvorrichtung kann es sich beispielsweise um eine Vorrichtung zur Hämodialyse (HD) oder Hämofiltration (HF) sowie eine Vorrichtung handeln, die sowohl eine Hämodialyse als auch Hämofiltration erlaubt (Hämo(dia)filtrationsvorrichtung). Die Blutbehandlungsvorrichtung kann daher über einen Dialysator oder Filter verfügen.

Nachfolgend wird eine Blutbehandlungsvorrichtung beschrieben, die über die Vorrichtung zur Überwachung des Flüssigkeitssystems verfügt. Dies wird in der Praxis der Fall sein, da die Vorrichtung zur Überwachung des Flüssigkeitssystems von einzelnen Komponenten Gebrauch macht, die in der Blutbehandlungsvorrichtung ohnehin vorhanden sind. Es ist aber auch möglich, dass die Überwachungsvorrichtung eine selbstständige Einheit bildet.

Die extrakorporale Blutbehandlungsvorrichtung , insbesondere Hämo(dia)filtrationsvorrichtung, weist einen Dialysator 1 oder Filter auf, der durch eine semipermeable Membran 2 in eine erste Kammer 3 und eine zweite Kammer 4 unterteilt ist. Von dem Patienten führt eine arterielle Blutleitung 5 zu dem Einlass 3A der ersten Kammer 3 des Dialysators 1. Von dem Auslass 3B der Blutkammer 3 geht eine venöse Blutleitung 6 ab, die zu dem Patienten führt. Zum Fördern des Bluts ist in der arteriellen Blutleitung 4 eine peristaltische Blutpumpe 7 angeordnet. In der venösen Blutleitung 6 ist stromab der Blutkammer 3 eine venöse Tropfkammer 8 angeordnet, die verhindert, dass Luft in den Patienten gelangt. Die venöse Tropfkammer 8 wird während der extrakorporalen Blutbehandlung von Blut durchflossen. Im Vorfeld oder nach der Behandlung kann die Tropfkammer von einer Ersatzflüssigkeit, insbesondere einer Kochsalzlösung, durchflossen werden.

Das Blut aus der Blutkammer 3 fließt über einen ersten Leitungsabschnitt 6A der venösen Blutleitung 6 zu einem Einlass 8A der venösen Tropfkammer 8, der am Deckel der Tropfkammer 8 angeordnet ist. Von einem Auslass 8B am Boden der venösen Tropfkammer 8 fließt das Blut über einen Leitungsabschnitt 6B der venösen Blutleitung 6 zum Patienten. In der Tropfkammer bildet sich ein Flüssigkeitspegel 9 aus. Die Höhe des Flüssigkeitspegels 9, insbesondere Blutpegels, wird bei dem vorliegenden Ausführungsbeispiel mit einer Einrichtung 10 überwacht. Es ist aber auch möglich, mit der Einrichtung nur zu überwachen, ob die Tropfkammer mit Blut gefüllt ist, um ein Leerlaufen der Tropfkammer auszuschließen, wie im Stand der Technik bekannt ist. Stromab der venösen Tropfkammer 8 ist in dem Leitungsabschnitt 6B der venösen Blutleitung 6 ein venöses Absperrorgan 11, insbesondere eine elektromagnetisch betätigbare Schlauchklemme angeordnet, die bei einem Störfall geschlossen werden kann, so dass kein Blut mehr zu dem Patienten gelangt. Von einem zweiten Auslass 8C am Deckel der Tropfkammer 8 zweigt eine Belüftungsleitung 12 ab, die zu einem elektromagnetisch betätigbaren Drei-Wege-Ventil 13 führt. An den einen Zweig 13A des Drei-Wege-Ventils 13 ist eine Messeinheit 14 zum Messen des Drucks in der Belüftungsleitung 12 angeschlossen, während an den anderen Zweig 13B ein Druckminderer 15 angeschlossen ist.

Zwischen der venösen Tropfkammer 8 und dem Drei-Wege-Ventil 13 sind in der Belüftungsleitung 12 zwei weitere hydrophobe Filter 16, 17 angeordnet, von denen der erste hydrophobe Filter 16 außerhalb des nicht dargestellten Gehäuses der Blutbehandlungsvorrichtung und der zweite Hydrophobfilter 17 innerhalb des Gehäuses der Blutbehandlungsvorrichtung angeordnet ist, so dass der erste Hydrophobfilter 16 noch relativ leicht, der zweite Hydrophobfilter 17 aber nur relativ schwierig zugänglich ist. Beide Hydrophobfilter 16, 17 weisen wieder eine hydrophobe Membran 16A, 17A auf.

Die extrakorporale Blutbehandlungsvorrichtung verfügt über eine zentrale Steuer- und Recheneinheit 18, mit der die einzelnen Komponenten der Blutbehandlungsvorrichtung nach den Vorgaben des Benutzers gesteuert werden. Bei dem vorliegenden Ausführungsbeispiel umfasst die zentrale Steuer- und Recheneinheit 18 der Blutbehandlungsvorrichtung auch die Steuereinheit bzw. die Steuer- und Recheneinheit der Vorrichtung zur Überwachung des Flüssigkeitssystems. Die Überwachungsvorrichtung kann aber auch über eine separate Steuereinheit bzw. Steuer- und Recheneinheit verfügen. Die zentrale Steuer- und Recheneinheit 18 ist optional über eine Steuerleitung 7A mit der Blutpumpe 7, eine Steuerleitung 11A mit der venösen Schlauchklemme 11 und eine Steuerleitung 13C mit dem Drei-Wege-Ventil 13 verbunden. Über eine Datenleitung 10A ist die Steuer- und Recheneinheit 18 mit der Überwachungseinrichtung 10 für den Blutpegel und eine Datenleitung 14A mit der Messeinheit 14 zum Messen des Drucks in der Belüftungsleitung 12 verbunden.

Darüber hinaus ist eine Alarmeinheit 19 vorgesehen, die einen akustischen und/oder optischen Alarm gibt. Die Alarmeinheit 19 ist über eine Datenleitung 19A mit der Steuer- und Recheneinheit 18 verbunden ist.

Neben dem extrakorporalen Blutkreislauf I weist die extrakorporale Blutbehandlungsvorrichtung einen Dialysierflüssigkeitskreislauf II auf, der nur andeutungsweise dargestellt ist. Er weist eine Dialysierflüssigkeitszuführleitung 20 die zu dem Einlass 4A der zweiten Kammer 4 des Dialysators 1 führt und eine Dialysierflüssigkeitsabführleitung 21 auf, die von dem Auslass 4B der Dialysierflüssigkeitskammer abgeht.

Zum Befüllen des extrakorporalen Blutkreislaufs I steuert die zentrale Steuer- und Recheneinheit 18 das Drei-Wege-Ventil 13 derart an, dass eine Verbindung zu dem Druckminderer 15 zum Belüften des Systems hergestellt ist. Folglich kann Luft aus der venösen Tropfkammer 8 über die Belüftungsleitung 12 und den Druckminderer 15 entweichen. Während der Blutbehandlung hingegen steuert die Steuer- und Recheneinheit 18 das Drei-Wege-Ventil 13 derart an, dass eine Verbindung zu der Druckmesseinheit 14 hergestellt ist. Damit ist die Belüftungsleitung am Ende verschlossen.

Wenn die venöse Schlauchklemme 11 geschlossen ist und die Blutpumpe 7 betrieben wird, steigt der Blutpegel 9 in der venösen Tropfkammer 8 an, wodurch sich in der Kammer 8A und der davon abzweigenden Belüftungsleitung 12, die am Ende verschlossen ist, ein Druck aufbaut, der mit der Messeinheit 14 erfasst werden kann.

Zunächst wird die Überwachungseinrichtung 10 für den Flüssigkeitspegel beim Überschreiten eines vorgegebenen oberen Blutpegels in der venösen Tropfkammer 8 ansprechen, die über die Datenleitung 10A mit der Steuer- und Recheneinheit 18 verbunden ist. Die Überwachungseinrichtung 10 erzeugt dann ein Steuersignal, das die Steuer- und Recheneinheit 18 empfängt.

Die Steuer- und Recheneinheit 18 umfasst eine Einheit 18A, die bei dem Empfang des Steuersignals der Überwachungseinrichtung 10 ein Steuersignal für einen Eingriff in die Maschinensteuerung erzeugt. Daraufhin wird die Blutpumpe 7 angehalten, so dass der Blutpegel 9 nicht weiter ansteigt.

Die erfindungsgemäße Vorrichtung zur Überwachung des Flüssigkeitssystems stellt bei dem vorliegenden Ausführungsbeispiel ein redundantes System dar, dass nur dann zur Anwendung kommt, wenn die Überwachungseinrichtung 10 für den Flüssigkeitspegel versagt. Die erfindungsgemäße Vorrichtung zur Überwachung des Flüssigkeitssystems kann auch dann als ein redundantes System eingesetzt werden, wenn mit der Überwachungseinrichtung nur das Vorhandensein von Blut in der venösen Tropfkammer überwacht wird. Die erfindungsgemäße Vorrichtung kann aber auch von der Überwachungseinrichtung völlig unabhängig arbeiten. Auch wenn im vorliegend Fall von Blut die Rede ist, kann die erfindungsgemäße Vorrichtung im Vorfeld oder nach der Behandlung die Benetzung oder Zerstörung der Membran eines Hydrophobfilters auch durch eine Ersatzflüssigkeit verhindern.

Eine erste Ausführungsform der erfindungsgemäßen Überwachungsvorrichtung sieht vor, dass die zentrale Steuer- und Recheneinheit 18 das venöse Absperrorgan 11 für ein vorgegebenes erstes Zeitintervall zumindest teilweise schließt, um die venöse Schlauchklemme 11 dann wieder für ein vorgegebenes zweites Zeitintervall, das mit dem ersten Zeitintervall identisch sein kann, vollständig zu öffnen. Das Öffnen und Schließen der Schlauchklemme erfolgt alternierend. Da die bekannten Schlauchklemmen, die in der Praxis zum Einsatz kommen insbesondere aus Sicherheitsgründen nur einen vollständig geöffneten und geschlossenen Zustand zulassen, wird der Blutfluss in der Praxis vollständig unterbrochen werden müssen. Grundsätzlich ist es aber auch möglich, die venöse Blutleitung 6 nur teilweise zu verschließen.

Das alternierende Öffnen und Schließen der venösen Schlauchklemme 11 erzeugt in dem Flüssigkeitssystem der Blutbehandlungsvorrichtung, das die Belüftungsleitung 12 umfasst, periodische Druckschwankungen, die mit der Druckmesseinheit 14 gemessen werden. Die Steuer- und Recheneinheit 18 wertet das gemessene Drucksignal aus, um eine Störung erkennen zu können. Dabei wird auf eine Störung dann geschlossen, wenn die Druckschwankungen ausbleiben. Das Ausbleiben von Druckschwankungen ist damit zu erklären, dass die hydrophobe Membran 16A des ersten Filters 16 der beiden in Reihe angeordneten Hydrophobfilter 16, 17 in einem Störfall mit Blut benetzt wird. Dadurch ist die Hydrophobmembran 16A auch für Luft nicht mehr durchlässig. Folglich können die im extrakorporalen Blutkreislauf I erzeugten Druckschwankungen in der Belüftungsleitung 12 stromab des Hydrophobfilters 16 mit der Druckmesseinheit 14 nicht mehr nachgewiesen werden.

Eine besonders bevorzugte alternative Ausführungsform sieht vor, dass die periodischen Druckschwankungen in dem Flüssigkeitssystem nicht durch die venöse Schlauchklemme 11 erzeugt werden, sondern dadurch, dass die Belüftungsleitung 12 bei zumindest teilweise geschlossenem venösen Absperrorgan 11 für ein vorgegebenes Zeitintervall belüftet wird, wenn die Blutpumpe 7 betrieben wird. Bei laufender Blutpumpe 7 und geschlossener venöser Schlauchklemme 11 steigt der Blutpegel 9 in der venösen Tropfkammer 8 stetig an, wodurch wiederum der Druck in der am Ende verschlossenen Belüftungsleitung 12 ansteigt. Eine Belüftung der Belüftungsleitung führt daher zu einem Druckabfall in der Belüftungsleitung und der venösen Tropfkammer sowie zu einem Absinken des Blutpegels in der Tropfkammer.

Die Steuer- und Recheneinheit 18 steuert das venöse Absperrorgan 11 derart an, dass das Absperrorgan 11 für ein vorgegebenes Zeitintervall geschlossen wird. In diesem vorgegeben Zeitintervall $\Delta t$ steuert die Steuer- und Recheneinheit 18 das Drei-Wege-Ventil 13 dann derart an, dass die Verbindung zu dem Druckminderer 15 alternierend geöffnet oder verschlossen wird, so dass die Belüftungsleitung zeitweise belüftet wird. Vorzugsweise erfolgt das alternierende Öffnen und Schließen des Belüftungsventils 13 synchronisiert mit der Rotation der peristaltischen Blutpumpe 7, mit der das Volumen an Luft komprimiert wird.

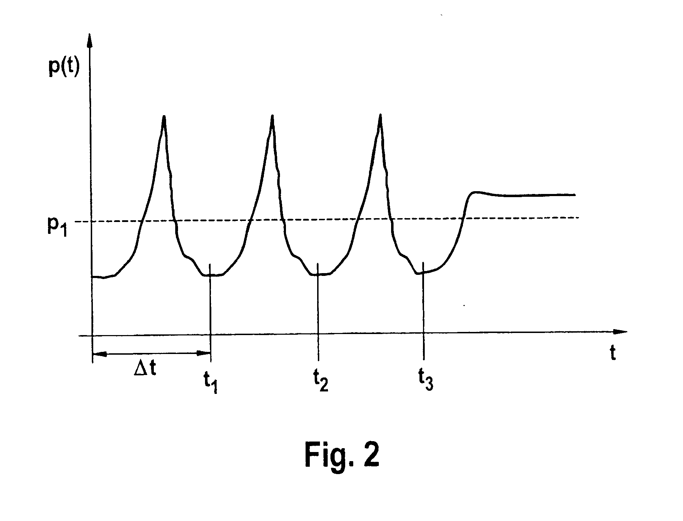

Fig. 2 zeigt das mit der Druckmesseinheit 14 gemessene Drucksignal p(t) als Funktion der Zeit t. Es zeigt sich, dass der Druck bei laufender Blutpumpe 7 und geschlossenem venösem Absperrorgan 11 zunächst ansteigt, um dann nach Ablauf des Zeitintervalls $\Delta t$ zum Zeitpunkt $t_1$ wieder auf den ursprünglichen Wert abzufallen. Dieser Vorgang wiederholt sich dann periodisch, so dass ein oszillierendes Drucksignal p(t) erzeugt wird, das mit der Messeinheit 14 gemessen wird.

Für den Fall, dass die Membran 16A des ersten Filters 16 der beiden in Reihe geschalteten Hydrophobfilter 16, 17 mit Blut benetzt werden sollte, so dass der Filter nunmehr auch für Luft undurchlässig wird, kann sich der Druck in der Belüftungsleitung 12 nicht mehr auf den ursprünglichen Wert aufbauen. Folglich kann bei fehlender Oszillation des Drucksignals p(t) sicher auf einen Störfall geschlossen werden.

Die zentrale Steuer- und Recheneinheit 18 überwacht während der Druckmessung das Drucksignal p(t), wobei überprüft wird, ob nach jeder Belüftung, d.h. nach Schließen des Belüftungsventils 13, ein vorgegebener Druckanstieg oder Druckabfall feststellbar ist. Beispielsweise überprüft die Steuer- und Recheneinheit 18, ob der Druck über einen vorgegebenen Grenzwert $p_1$ ansteigt. Dies ist bei dem Ausführungsbeispiel nach der dritten Periode der Fall. Die Auswertung erlaubt die Erkennung der Benetzung der hydrophoben Membran in einem so kurzen Zeitraum, dass nur wenige Milliliter Volumen an Blut gefördert werden, bevor die Steuer- und Recheneinheit 18 durch Stoppen der Blutpumpe 7 einen Eingriff in die Maschinensteuerung vorgenommen hat. Anstelle der Überwachung eines vorgegebenen Grenzwerts $p_1$ für das Drucksignal p(t) kann auch die Steigung des Drucksignals p(t) mit der Steuer- und Recheneinheit 18 berechnet und überwacht werden. Ein Eingriff in die Maschinensteuerung wird dann vorgenommen, wenn die Steigung des Drucksignals p(t) nach Schließen des Belüftungsventils 13 einen vorgegebenen Grenzwert unterschreitet, d.h. ein signifikanter Anstieg des Drucksignals nicht mehr zu verzeichnen ist.

Anhand des Steigungsverlaufs des Drucksignals p(t) kann insbesondere während des geschlossenen Belüftungsventils auf die Rest-Volumenmenge in dem Flüssigkeitssystem geschlossen werden. Aus der allgemeinen Gasgleichung folgt bei isothermischen Bedingungen der Zusammenhang nach Boyle und Mariotte, dass die Volumenabnahme und die Druckzunahme im reziproken Zusammenhang stehen:

$$\Delta V \propto \frac{1}{\Delta p}$$

Mit der obigen Beziehung lässt sich anhand des absoluten Druckes p gegenüber $p_0$ das komprimierte Restvolumen V gegenüber $V_0$ abschätzen. Hierbei sind die Randbedingungen durch $V_0 \cdot p_0 = c$ gegeben. Die Konstante c beschreibt die Compliance des pneumatischen Systems.

$$V = V_0 + \frac{\partial V}{\partial p}(p_0 + p) = V_0 - \frac{c}{p_0 + p}$$

Da sowohl die Schlauchleitungen als auch die Tropfkammer herstellungsbedingten Toleranzen unterliegen, sind Veränderungen des in den entsprechenden Schlauchleitungen und der Tropfkammer eingeschlossenen Luftvolumens zu erwarten. Hinzu kommen Schwankungen der Umgebungstemperatur. Dies kann sich in der Praxis bei der Auswertung der Messwerte als problematisch erweisen.

Aus dem idealen Gasgesetz p V = n RT ist ersichtlich, dass bei identischem Blutpegel der detektierte Druck sich allein aufgrund der Variationen des eingeschlossenen Luftvolumens verändern kann. Daher ist eine Kalibrierung der erfindungsgemäßen Messvorrichtung vorgesehen, die durch das Bedienpersonal erfolgen kann, die ein entsprechendes Kalibrierprogramm startet. Hierzu wird zunächst das Schlauchset mit der arteriellen und venösen Blutleitung 5, 6 bei geschlossener venöser Schlauchklemme 11 befüllt. Bei langsam laufender Blutpumpe 7 wird der Blutspiegel 9 in der Tropfkammer 8 beobachtet. Wenn der Blutspiegel einen kritischen oberen Grenzwert erreicht, gibt die bedienende Person den oberen Grenzwert als den Grenzwert vor, bei dem die Blutpumpe 7 gestoppt und die Schlauchklemme 11 geöffnet wird. Diese Vorgabe kann durch eine entsprechende Interaktion mit der Maschine, beispielsweise durch Drücken einer Taste erfolgen. Die Temperatur kann für die Dauer der Behandlung als hinreichend konstant angesehen werden. Es ist aber auch möglich, die Temperatur mit Temperatursensoren zu erfassen und bei der Auswertung des gemessenen Drucksignals auch Temperaturänderungen zu berücksichtigen.

Es ist grundsätzlich möglich, Druckschwankungen in der venösen Tropfkammer mit einem Kompressor zu erzeugen, wobei sich der Flüssigkeitspegel dann in der venösen Tropfkammer wegen der zu vernachlässigenden Kompressibilität von Flüssigkeiten nicht ändert. Hierzu kann der Kompressor über eine zusätzliche Belüftungsleitung, in der mindestens ein zusätzlicher hydrophober Filter angeordnet ist, an das System angeschlossen werden. Mit dem Kompressor kann die Luft in dem System dann alternierend verdichtet bzw. entspannt werden. Eine Benetzung der Membran des zu überprüfenden Hydrophobfilters hätte dann zur Folge, dass keine Druckänderungen mehr detektiert werden können, so dass auf einen Störfall geschlossen werden kann.

**Patentansprüche**

1. Vorrichtung zur Überwachung eines Flüssigkeitssystems einer extrakorporalen Blutbehandlungsvorrichtung, wobei die extrakorporale Blutbehandlungsvorrichtung aufweist:

einen extrakorporalen Blutkreislauf (I), der eine arterielle Blutleitung (5), die zu einer ersten Kammer (3) eines durch eine semipermeable Membran (2) in die erste Kammer (3) und eine zweite Kammer (4) unterteilten Dialysators (1) oder Filters führt, und eine venöse Blutleitung (6) aufweist, die von der ersten Kammer (3) des Dialysators (1) oder Filters abgeht, wobei in der venösen Blutleitung (5) stromab der ersten Kammer (3) des Dialysators (1) oder Filters eine venöse Tropfkammer (8) angeordnet ist, und stromab der venösen Tropfkammer (8) in der venösen Blutleitung ein venöses Absperrorgan (11) angeordnet ist, eine im extrakorporalen Blutkreislauf angeordnete Blutpumpe (7), und eine von der venösen Tropfkammer (8) abzweigenden Belüftungsleitung (12), in der mindestens ein hydrophober Filter (16) angeordnet ist, wobei die Belüftungsleitung (12) mit einem Belüftungsventil (13) verschließbar ist, das in dem der venösen Tropfkammer abgewandten Abschnitt der Belüftungsleitung angeordnet ist, wobei die Vorrichtung zur Überwachung des Flüssigkeitssystems aufweist: eine Messeinheit (14) zum Messen des Drucks in der von der venösen Tropfkammer abzweigenden Belüftungsleitung in dem der venösen Tropfkammer abgewandten Abschnitt der Belüftungsleitung, und eine Steuer- und Recheneinheit (18) zum Auswerten des gemessenen Drucksignals, wobei die Steuer- und Recheneinheit (18) derart ausgebildet ist, dass auf eine Störung geschlossen wird, wenn Druckschwankungen des Drucksignals ausbleiben **dadurch gekennzeichnet, dass** die Vorrichtung zur Überwachung des Flüssigkeitssystems eine Steuereinheit (18) für das venöse Absperrorgan (11) aufweist, die derart ausgebildet ist, dass das venöse Absperrorgan alternierend zumindest teilweise geöffnet und geschlossen wird, so dass der Flüssigkeitspegel (9) in der venösen Tropfkammer (8) alternierend ansteigt und absinkt, wodurch das gemessene Drucksignal periodischen Druckschwankungen unterworfen ist, wenn eine Störung nicht vorliegt.

2. Vorrichtung zur Überwachung eines Flüssigkeitssystems einer extrakorporalen Blutbehandlungsvorrichtung, wobei die extrakorporale Blutbehandlungsvorrichtung aufweist:

einen extrakorporalen Blutkreislauf (I), der eine arterielle Blutleitung (5), die zu einer ersten Kammer (3) eines durch eine semipermeable Membran (2) in die erste Kammer (3) und eine zweite Kammer (4) unterteilten Dialysators (1) oder Filters führt, und eine venöse Blutleitung (6) aufweist, die von der ersten Kammer (3) des Dialysators (1) oder Filters abgeht, wobei in der venösen Blutleitung (5) stromab der ersten Kammer (3) des Dialysators (1) oder Filters eine venöse Tropfkammer (8) angeordnet ist, und stromab der venösen Tropfkammer (8) in der venösen Blutleitung ein venöses Absperrorgan (11) angeordnet ist, eine im extrakorporalen Blutkreislauf angeordnete Blutpumpe (7), und eine von der venösen Tropfkammer (8) abzweigenden Belüftungsleitung (12), in der mindestens ein hydrophober Filter (16) angeordnet ist, wobei die Belüftungsleitung (12) mit einem Belüftungsventil (13) verschließbar ist, das in dem der venösen Tropfkammer abgewandten Abschnitt der Belüftungsleitung angeordnet ist, wobei die Vorrichtung zur Überwachung eines Flüssigkeitssystems aufweist:

eine Messeinheit (14) zum Messen des Drucks in der von der venösen Tropfkammer abzweigenden Belüftungsleitung in dem der venösen Tropfkammer abgewandten Abschnitt der Belüftungsleitung, und eine Steuer- und Recheneinheit (18) zum Auswerten des gemessenen Drucksignals, wobei die Rechen- und Steuereinheit (18) derart ausgebildet ist, dass auf eine Störung geschlossen wird, wenn periodische Druckschwankungen des Drucksignals ausbleiben, **dadurch gekennzeichnet, dass** die Vorrichtung zur Überwachung des Flüssigkeitssystems eine Steuereinheit (18) für das Belüftungsventil (13) aufweist, die derart ausgebildet ist, dass bei zumindest teilweise geschlossenem venösen Absperrorgan (11) das Belüftungsventil (13) alternierend zumindest teilweise geöffnet und geschlossen wird, so dass der Flüssigkeitspegel (9) in der venösen Tropfkammer (8) alternierend ansteigt und absinkt und das gemessene Drucksignal periodischen Druckschwankungen unterworfen ist, wenn eine Störung nicht vorliegt.

3. Vorrichtung nach Anspruch 1 oder 2, **dadurch gekennzeichnet, dass** die Steuer- und Recheneinheit (18) derart ausgebildet ist, dass überprüft wird, ob ein vorgegebener Druckanstieg feststellbar ist,

4. Vorrichtung nach einem der Ansprüche 1 bis 3, **dadurch gekennzeichnet, dass** die Steuer- und Recheneinheit (18) derart ausgebildet ist, dass überprüft wird, ob der Druck über einen vorgegebenen Grenzwert ansteigt.

5. Vorrichtung nach einem der Ansprüche 1 bis 4, **dadurch gekennzeichnet, dass** eine Alarmeinheit (19) zum Erzeugen eines akustischen und/oder optischen Alarms vorgesehen ist, die derart mit der Steuer- und Recheneinheit (18) zusammenwirkt, dass ein akustischer und/oder optischer Alarm erzeugt wird, wenn eine Störung festgestellt wird.

6. Vorrichtung nach einem der Ansprüche 1 bis 5, **dadurch gekennzeichnet, dass** eine Einheit (18A) zum Erzeugen eines Steuersignals für einen Eingriff in die Steuerung der extrakorporalen Blutbehandlungsvorrichtung vorgesehen ist, die derart mit der Steuer- und Recheneinheit (18) zusammenwirkt, dass das Steuersignal erzeugt wird, wenn eine Störung festgestellt wird.

7. Vorrichtung nach Anspruch 6, **dadurch gekennzeichnet, dass** die Einheit (18A) zum Erzeugen eines Steuersignals für einen Eingriff in die Steuerung der extrakorporalen Blutbehandlungsvorrichtung derart ausgebildet ist, dass als Eingriff in die Maschinensteuerung die Blutpumpe (7) angehalten wird, wenn eine Störung festgestellt wird.

8. Extrakorporale Blutbehandlungsvorrichtung mit
einem extrakorporalen Blutkreislauf (I), der eine arterielle Blutleitung (5), die zu einer ersten Kammer (3) eines durch eine semipermeable Membran (2) in die erste Kammer (3) und eine zweite Kammer (4) unterteilten Dialysators (1) oder Filters führt, und eine venöse Blutleitung (6) aufweist, die von der ersten Kammer (3) des Dialysators (1) oder Filters abgeht, wobei in der venösen Blutleitung (5) stromab der ersten Kammer (3) des Dialysators (1) oder Filters eine venöse Tropfkammer (8) angeordnet ist, und stromab der venösen Tropfkammer in der venösen Blutleitung ein venöses Absperrorgan (11) angeordnet ist,
einer im extrakorporalen Blutkreislauf angeordneten Blutpumpe (7),
einer von der venösen Tropfkammer (8) abzweigenden Belüftungsleitung (12), in der mindestens ein hydrophober Filter (16) angeordnet ist, wobei die Belüftungsleitung (12) mit einem Belüftungsventil (13) verschließbar, das in dem der venösen Tropfkammer abgewandten Abschnitt der Belüftungsleitung angeordnet ist,
**dadurch gekennzeichnet, dass** die extrakorporale Blutbehandlungsvorrichtung eine Vorrichtung zur Überwachung des Flüssigkeitssystems der extrakorporalen Blutbehandlungsvorrichtung nach einem der Ansprüche 1 bis 7 aufweist.

**Claims**

1. Apparatus for monitoring a fluid system of an extracorporeal blood treatment apparatus, wherein the extracorporeal blood treatment apparatus has:

an extracorporeal blood circuit (I) that has an arterial blood line (5) that leads to a first chamber (3) of a dialyzer (1) or filter divided into the first chamber (3) and a second chamber (6) by means of a semipermeable membrane (2), and a venous blood line that proceeds from the first chamber (3) of the dialyzer (1) or filter, wherein a venous drip chamber (8) is disposed in the venous blood line (5), downstream from the first chamber (3) of the dialyzer (1) or filter, and a venous shut-off device (11) is disposed in the venous blood line downstream from the venous drip chamber (8),
a blood pump (7) disposed in the extracorporeal blood circuit, and
a ventilation line (12) that branches off from the venous drip chamber (8), in which line at least one hydrophobic filter (16) is disposed, wherein the ventilation line (12) can be closed off using a ventilation valve (13) that is disposed in the section of the ventilation line that faces away from the drip chamber,
wherein the apparatus has the following for monitoring the fluid system:

a measuring unit (14) for measuring the pressure in the ventilation line that branches off from the venous drip chamber, in the section of the ventilation line that faces away from the venous drip chamber, and
a control and calculation unit (18) for evaluating the measured pressure signal, wherein the control and calculation unit (18) is configured in such a manner that a conclusion concerning a problem is drawn if pressure variations of the pressure signal fail to occur,
**characterized in that**
the apparatus for monitoring the fluid system has a control unit (18) for the venous shut-off device (11), which unit is configured in such a manner that the venous shut-off device is alternately opened and closed, at least in part, so that the fluid level (9) in the venous drip chamber (8) alternatively rises and drops, and thereby the measured pressure signal is subjected to periodic pressure variations if no problem exists.

2. Apparatus for monitoring a fluid system of an extracorporeal blood treatment apparatus, wherein the extracorporeal blood treatment apparatus has:

an extracorporeal blood circuit (I) that has an arterial blood line (5) that leads to a first chamber (3) of a dialyzer (1) or filter divided into the first chamber (3) and a second chamber (6) by means of a semipermeable membrane (2), and a venous blood line that proceeds from the first chamber (3) of the dialyzer (1) or filter, wherein a venous drip chamber (8) is disposed in the venous blood line (5), downstream from the first chamber (3) of the dialyzer (1) or filter, and a venous shut-off device (11) is disposed in the venous blood line downstream from the venous drip chamber (8),
a blood pump (7) disposed in the extracorporeal blood circuit, and
a ventilation line (12) that branches off from the venous drip chamber (8), in which line at least one hydrophobic filter (16) is disposed, wherein the ventilation line (12) can be closed off using a ventilation valve (13) that is disposed in the section of the ventilation line that faces away from the drip chamber,
wherein the apparatus has the following for monitoring the fluid system:

a measuring unit (14) for measuring the pressure in the ventilation line that branches off from the venous drip chamber, in the section of the ventilation line that faces away from the venous drip chamber, and
a control and calculation unit (18) for evaluating the measured pressure signal, wherein the control and calculation unit (18) is configured in such a manner that a conclusion concerning a problem is drawn if periodic pressure variations of the pressure signal fail to occur,
**characterized in that**
the apparatus for monitoring the fluid system has a control unit (18) for the ventilation valve (13), which unit is configured in such a manner that when the venous shut-off device (11) is closed, at least in part, the ventilation valve (13) is alternately opened and closed, at least in part, so that the fluid level (9) in the venous drip chamber (8) alternatively rises and drops, and the measured pressure signal is subjected to periodic pressure variations if no problem exists.

3. Apparatus according to claim 1 or 2, **characterized in that** the control and calculation unit (18) is configured in such a manner that a check is conducted as to whether a predetermined pressure increase can be determined.

4. Apparatus according to one of claims 1 to 3, **characterized in that** the control and calculation unit (18) is configured in such a manner that a check is conducted as to whether the pressure rises above a predetermined limit value.

5. Apparatus according to one of claims 1 to 4, **characterized in that** an alarm unit (19) is provided for generating an acoustical and/or optical alarm, which unit interacts with the control and calculation unit (18) in such a manner that an acoustical and/or optical alarm is generated if a problem is found.

6. Apparatus according to one of claims 1 to 5, **characterized in that** a unit (18A) for generating a control signal for intervention in the control of the extracorporeal blood treatment apparatus is provided, which unit interacts with the control and calculation unit (18) in such a manner that a control signal is generated if a problem is found.

7. Apparatus according to claim 6, **characterized in that** the unit (18A) for generating a control signal for intervention in the control of the extracorporeal blood treatment apparatus is configured in such a manner that as an intervention in the machine control, the blood pump (7) is stopped if a problem is found.

8. Extracorporeal blood treatment apparatus, having
an extracorporeal blood circuit (I) that has an arterial blood line (5) that leads to a first chamber (3) of a dialyzer (1) or filter divided into the first chamber (3) and a second chamber (6) by means of a semipermeable membrane (2), and a venous blood line that proceeds from the first chamber (3) of the dialyzer (1) or filter, wherein a venous drip chamber (8) is disposed in the venous blood line (5), downstream from the first chamber (3) of the dialyzer (1) or filter, and a venous shut-off device (11) is disposed in the venous blood line downstream from the venous drip chamber (8),
a blood pump (7) disposed in the extracorporeal blood circuit,
a ventilation line (12) that branches off from the venous drip chamber (8), in which line at least one hydrophobic filter (16) is disposed, wherein the ventilation line (12) can be closed off using a ventilation valve (13) that is disposed in the section of the ventilation line that faces away from the drip chamber,
**characterized in that** the extracorporeal blood treatment apparatus has an apparatus for monitoring the fluid system of the extracorporeal treatment apparatus, according to one of claims 1 to 7.

## Revendications

1. Dispositif de surveillance d'un système à liquide d'un dispositif de traitement extracorporel du sang, dans lequel le dispositif extracorporel du sang présente :

   une circulation sanguine extracorporelle (I) qui présente une conduite de sang artériel (5) qui conduit à une première chambre (3) d'un dialyseur (1) ou d'un filtre subdivisé par une membrane semi-perméable (2) en la première chambre (3) et une deuxième chambre (4) et une conduite de sang veineux (6) qui part de la première chambre (3) du dialyseur (1) ou du filtre, dans lequel dans la conduite de sang veineux (5) en aval de la première chambre (3) du dialyseur (1) ou du filtre est disposée une chambre compte-goutte veineuse (8) et en aval de la chambre compte-goutte veineuse (8) est disposé, dans la conduite de sang veineux, un organe de blocage veineux (11),
   une pompe de sang (7) disposée dans la circulation sanguine extracorporelle, et une conduite de ventilation (12) dérivant de la chambre compte-goutte veineuse (8) dans laquelle au moins un filtre hydrophobe (16) est disposé, dans lequel la conduite de ventilation (12) peut être fermée avec une soupape de ventilation (13) qui est disposée dans la partie détournée de la chambre compte-goutte veineuse de la conduite de ventilation,
   dans lequel le dispositif de surveillance du système à liquide présente :

   une unité de mesure (14) pour mesurer la pression dans la conduite de ventilation dérivant de la chambre compte-goutte veineuse dans la partie détournée de la chambre compte-goutte veineuse de la conduite de ventilation, et
   une unité de commande et de calcul (18) pour analyser le signal de pression mesuré, dans lequel l'unité de commande et de calcul (18) est conçue de telle sorte qu'on en conclue à une perturbation, en l'absence de variations de pression du signal de pression,
   **caractérisé en ce que**
   le dispositif de surveillance du système à liquide présente une unité de commande (18) pour l'organe de blocage veineux (11) qui est conçue de telle sorte que l'organe de blocage veineux soit ouvert et fermé au moins partiellement en alternance, de sorte que le niveau de liquide (9) dans la chambre compte goutte veineuse (8) monte et descende en alternance, ce qui permet au signal de pression mesuré d'être soumis à des variations de pressions périodiques lorsqu'il n'existe aucune perturbation.

2. Dispositif de surveillance d'un système à liquide d'un dispositif de traitement extracorporel du sang, dans lequel le dispositif extracorporel du sang présente :

   une circulation sanguine extracorporelle (I) qui présente une conduite de sang artériel (5) qui conduit à une première chambre (3) d'un dialyseur (1) ou d'un filtre subdivisé par une membrane semi-perméable (2) en la première chambre (3) et une deuxième chambre (4) et une conduite de sang veineux (6) qui part de la première chambre (3) du dialyseur (1) ou du filtre, dans lequel dans la conduite de sang veineux (5) en aval de la première chambre (3) du dialyseur (1) ou du filtre est disposée une chambre compte-goutte veineuse (8) et en aval de la chambre compte-goutte veineuse (8) est disposé, dans la conduite de sang veineux, un organe de blocage veineux (11),
   une pompe de sang (7) disposée dans la circulation sanguine extracorporelle, et une conduite de ventilation (12) dérivant de la chambre compte-goutte veineuse (8) dans laquelle au moins un filtre hydrophobe (16) est disposé, dans lequel la conduite de ventilation (12) peut être fermée avec une soupape de ventilation (13) qui est disposée dans la partie détournée de la chambre compte-goutte veineuse de la conduite de ventilation,
   dans lequel le dispositif de surveillance du système à liquide présente :

   une unité de mesure (14) pour mesurer la pression dans la conduite de ventilation dérivant de la chambre compte-goutte veineuse dans la partie détournée de la chambre compte-goutte veineuse de la conduite de ventilation, et
   une unité de commande et de calcul (18) pour analyser le signal de pression mesuré, dans lequel l'unité de commande et de calcul (18) est conçue de telle sorte qu'on en conclue à une perturbation, en l'absence de variations de pression périodiques du signal de pression, **caractérisé en ce que**

le dispositif de surveillance du système à liquide présente une unité de commande (18) pour la soupape de ventilation (13) qui est conçue de telle sorte que lorsque l'organe de blocage veineux (11) est au moins partiellement fermé, la soupape de ventilation (13) est ouverte et fermée au moins partiellement en alternance, de sorte que le niveau de liquide (9) dans la chambre compte goutte veineuse (8) monte et descende en alternance, et le signal de pression mesuré est soumis à des variations de pressions périodiques lorsqu'il n'existe aucune perturbation.

3. Dispositif selon la revendication 1 ou 2, **caractérisé en ce que** l'unité de commande et de calcul (18) est conçue de telle sorte que l'on vérifie si une augmentation de pression prédéterminée peut être constatée.

4. Dispositif selon l'une des revendications 1 à 3, **caractérisé en ce que** l'unité de commande et de calcul (18) est conçue de telle sorte que l'on vérifie si la pression dépasse un seuil prédéterminé.

5. Dispositif selon l'une des revendications 1 à 4, **caractérisé en ce qu'**une unité d'alarme (19) pour générer une alarme acoustique et/ou optique est prévue, qui coopère avec l'unité de commande et de calcul (18) de telle sorte qu'une alarme acoustique et/ou optique soit générée lorsqu'une perturbation est constatée.

6. Dispositif selon l'une des revendications 1 à 5, **caractérisé en ce qu'**une unité (18A) pour générer un signal de commande pour une intervention dans la commande du dispositif de traitement extracorporel du sang est prévue, qui coopère avec l'unité de commande et de calcul (18) de telle sorte que le signal de commande soit généré lorsqu'une perturbation est constatée

7. Dispositif selon la revendication 6, **caractérisé en ce que** l'unité (18A) pour générer un signal de commande pour une intervention dans la commande du dispositif de traitement extracorporel du sang est conçue de telle sorte que, comme intervention dans la commande de la machine, la pompe de sang (7) est arrêtée lorsqu'une perturbation est constatée.

8. Dispositif de traitement extracorporel du sang comportant
une circulation sanguine extracorporelle (I) qui présente une conduite de sang artériel (5) qui conduit à une première chambre (3) d'un dialyseur (1) ou d'un filtre subdivisé par une membrane semi-perméable (2) en la première chambre (3) et une deuxième chambre (4) et une conduite de sang veineux (6) qui part de la première chambre (3) du dialyseur (1) ou du filtre, dans lequel dans la conduite de sang veineux (5) en aval de la première chambre (3) du dialyseur (1) ou du filtre est disposée une chambre compte-goutte veineuse (8) et en aval de la chambre compte-goutte veineuse (8) est disposé, dans la conduite de sang veineux, un organe de blocage veineux (11),
une pompe de sang (7) disposée dans la circulation sanguine extracorporelle, et
une conduite de ventilation (12) dérivant de la chambre compte-goutte veineuse (8) dans laquelle au moins un filtre hydrophobe (16) est disposé, dans lequel la conduite de ventilation (12) peut être fermée avec une soupape de ventilation (13) qui est disposée dans la partie détournée de la chambre compte-goutte veineuse de la conduite de ventilation,
**caractérisé en ce que** le dispositif de traitement extracorporel du sang présente un dispositif de surveillance du système à liquide du dispositif de traitement extracorporel du sang selon l'une des revendications 1 à 7.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0330761 A1